(19) Europäisches Patentamt
European Patent Office
Office européen des brevets

(11) Numéro de publication : **0 376 820 B1**

# (12) FASCICULE DE BREVET EUROPEEN

(45) Date de publication du fascicule du brevet : **03.03.93 Bulletin 93/09**

(51) Int. Cl.$^5$ : **A61K 7/15**, A61K 7/06

(21) Numéro de dépôt : **89403615.1**

(22) Date de dépôt : **22.12.89**

## (54) Composition de rasage pour la peau à base de polyorganosiloxanes à fonction acyloxyalkyle et procédé de mise en oeuvre.

(30) Priorité : **29.12.88 FR 8817433**

(43) Date de publication de la demande : **04.07.90 Bulletin 90/27**

(45) Mention de la délivrance du brevet : **03.03.93 Bulletin 93/09**

(84) Etats contractants désignés : **AT BE CH DE ES FR GB GR IT LI NL SE**

(56) Documents cités :
**EP-A- 0 219 830**
**DE-A- 1 467 863**
**GB-A- 2 066 659**
**US-A- 3 136 696**

(73) Titulaire : **L'OREAL**
**14, Rue Royale**
**F-75008 Paris (FR)**

(72) Inventeur : **Grollier, Jean-François**
**16bis boulevard Morland**
**F-75004 Paris (FR)**
Inventeur : **Caudet, Alain**
**255 boulevard Jean-Jaurès**
**F-92100 Boulogne-Billancourt (FR)**

(74) Mandataire : **Casalonga, Axel et al**
**BUREAU D.A. CASALONGA - JOSSE**
**Morassistrasse 8**
**W-8000 München 5 (DE)**

**EP 0 376 820 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouvelles compositions destinées au rasage de la peau à base de polyorganosiloxanes à fonction acyloxyalkyle et leur procédé de mise en oeuvre.

Certains polyorganosiloxanes sont bien connus dans le domaine des compositions de rasage et sont utilisés dans les formulations de crèmes à raser, de lotions avant rasage et de mousses à raser. Il s'agit principalement des silicones volatiles telles que le polydiméthylsiloxane ou "diméthicone" ou le polydiméthylcyclosiloxane ou "cyclométhicone".

Ces silicones, utilisées dans les compositions à raser, ont pour fonction d'atténuer, par leur pouvoir lubrifiant, le "feu" du rasoir, de rendre la peau plus douce et plus satinée et de conférer aux compositions qui les renferment un certain confort.

Cependant, ces silicones sont également connues pour leur propriété antimoussante et sont difficilement utilisables dans les mousses à raser conditionnées en aérosol. Lorsque ces silicones permettent la formation d'une mousse, celle-ci présente généralement une qualité et une stabilité très variables suivant le degré de remplissage du récipient conditionné en aérosol.

On connait dans l'état de la technique, des compositions de rasage à base de mélanges fluides de diméthylpolysiloxane et de méthylphénylpolysiloxane dans un milieu alcoolique. Elles sont décrites dans le brevet USP 3,136,696.

Des crèmes à raser à base d'organopolysiloxanes fluides ont été décrites dans la demande DE 1 467 863.

La demanderesse a découvert, d'une façon surprenante, que l'utilisation de polyorganosiloxanes à fonction acyloxyalkyle dans des compositions à raser, permettait d'améliorer sensiblement l'onctuosité et la douceur de ces compositions en plus des propriétés indiquées ci-dessus pour les silicones.

La demanderesse a découvert également que l'utilisation de ces polyorganosiloxanes à fonction acyloxyalkyle dans les mousses à raser permettait à celles-ci de conserver les qualités mentionnées ci-dessus et leur stabilité au cours de la vidange progressive du récipient conditionné en aérosol.

Enfin, la demanderesse a découvert que les compositions conformes à l'invention contenant des polyorganosiloxanes à fonction acyloalkyle, amélioraient la coupe du poil au cours du rasage, s'éliminaient remarquablement bien au rinçage à l'eau, aussi bien que la peau des lames de rasoir, et laissaient la peau rapidement nette et satinée.

Un objet de l'invention est constitué par les compositions destinées au rasage de la peau, contenant les polyorganosiloxanes à fonction acyloxyalkyle.

L'invention a également pour objet un procédé de rasage de la peau mettant en oeuvre ces compositions.

D'autres objets de l'invention apparaîtront à la lecture de la description et des exemples qui suivent.

La présente invention a donc pour objet une composition destinée au rasage de la peau, contenant dans un milieu aqueux cosmétiquement acceptable un agent moussant et au moins un polyorganosiloxane à fonction acyloxyalkyle choisi :

(i) parmi les composés linéaires répondant à la formule moyenne (I) suivante :

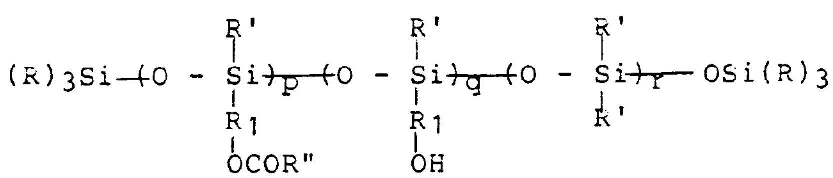

(I)

dans laquelle :

- les radicaux R, identiques ou différents, sont choisis parmi les radicaux méthyle, phényle, OCOR″, hydroxyle, un seul des R par atome de silicium peut être OH ;
- les radicaux R′, identiques ou différents, sont choisis parmi les radicaux méthyle et phényle, 60% molaire au moins de l'ensemble des radicaux R et R′ est méthyle ;
- $R_1$ est un chaînon alkylène divalent hydrocarboné, linéaire ou ramifié, comportant de 2 à 18 atomes de carbone, de préférence de 2 à 6 et plus particulièrement une chaîne triméthylène $-(CH_2)_3-$ et méthyl-2 triméthylène

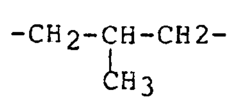

;

- R″ est un radical alcoyle ou alcényle en $C_8$-$C_{20}$ ;

- r est un nombre compris entre 1 et 120 inclus ;
- p est un nombre compris entre 1 et 30 ;
- q est égal à 0 ou est un nombre inférieur ou égal à 0,5 p, la somme p+q étant comprise entre 1 et 30.

(ii) les composés cycliques répondant à la formule moyenne suivante :

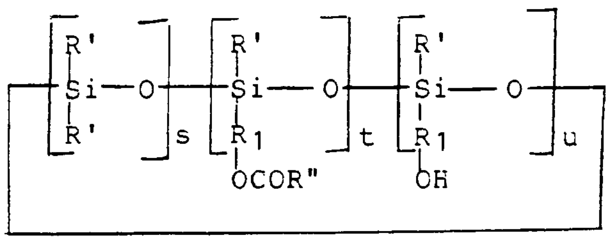

(II)

dans laquelle :

R′, R″, $R_1$ ont la même signification qu'indiquée ci-dessus ;

- s est un nombre compris entre 0 et 20 ;
- t est un nombre compris entre 1 et 20 ;
- u est égal à 0 ou est égal à un nombre inférieur ou égal à 0,5 t, la somme t+u étant comprise entre 1 et 20,

la somme s+t+u étant supérieure ou égale à 3.

Les polyorganosiloxanes à fonction acyloxyalkyle linéaire de formule (I) peuvent éventuellement comporter des groupements

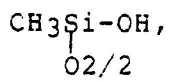

présents dans des proportions ne dépassant pas 15% de la somme p+q+r.

Parmi les composés particulièrement préférés, utilisés conformément à l'invention, on peut utiliser les composés de formule (I) ou (II), dans lesquelles :

R désigne un méthyle ;

R′ désigne un méthyle ;

$R_1$ désigne $-(CH_2)_3-$;

R″ désigne $C_{12}H_{25}$, $C_{14}H_{29}$, $C_{16}H_{33}$, $C_{18}H_{37}$, oléyle ;

r est compris entre 1 et 30 ;

p est compris entre 5 et 16 ;

q est compris entre 0,5 et 4 ;

s+t+u est compris entre 3 et 10 inclus.

Les composés de formules moyennes (I) et (II) sont obtenus par estérification de diorganopolysiloxanes à fonction hydroxyalkyle, de formules moyennes (III) et (IV) :

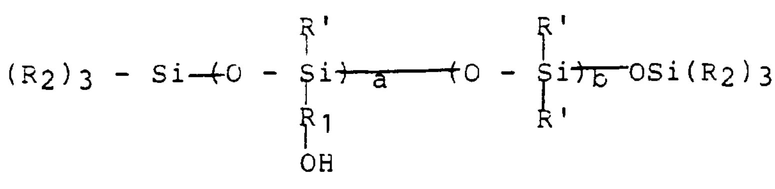

(III)

dans laquelle R″ et $R_1$ ont la même signification qu'indiquée ci-dessus ;

$R_2$ représente un radical méthyle, phényle ou hydroxyle, un seul des radicaux $R_2$ par atome de silicium peut être hydroxyle ;

60% au moins de l'ensemble des radicaux $R_2$ et R′ est méthyle ;

a est compris entre 1 et 30 et b est compris entre 1 et 20 ;

les composés de formule (III) pouvant comporter des motifs

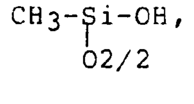

dans des proportions inférieures ou égales à 15% de la somme a+b.

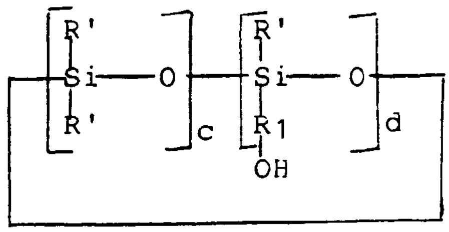

(IV)

dans laquelle R′, $R_1$ ont les mêmes significations que dans la formule (II) indiquée ci-dessus, c est compris entre 0 et 20 et d est compris entre 1 et 20, la somme c+d étant supérieure ou égale à 3.

L'estérification s'effectue de façon connue, avec un acide R″COOH ou l'anhydride d'acide, à une température comprise entre 100 et 250°C en présence éventuellement d'un catalyseur comme le chlorure d'aluminium ou le chlorure de zinc ou d'un acide fort comme l'acide chlorhydrique ou l'acide sulfurique.

On peut également effectuer une transestérification par chauffage à 100-150°C d'un ester méthylique de formule $R''COOCH_3$ et d'un diorganopolysiloxane de formule (III) ou (IV), en présence d'un catalyseur acide comme l'acide paratoluènesulfonique ou une terre acide de type Montmorillonite (KATALYSATOR KSF/O, vendu par SUD-CHEMIE - A.G. MUNCHEN).

Les produits de formules (III) et (IV) sont connus en eux-mêmes et peuvent être préparés suivant les procédés connus de l'art antérieur, tels que ceux décrits dans les brevets US-A-4.160.775, 2.970.150 ou dans la demande de brevet FR-85/16334 (EP-A-0 225 261).

Pour préparer les produits de formules (III) et (IV), on peut par exemple utiliser comme hydrogénopolysiloxane de départ, les copolymères de formules (V) ou (VI) :

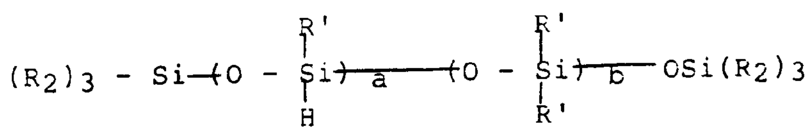

(V)

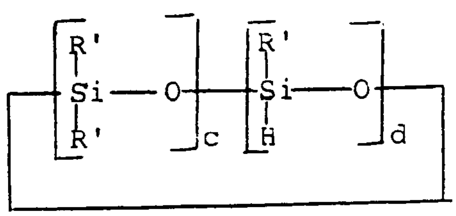

(VI)

dans lesquelles :

$R_2$, a, b, R′ ont les mêmes significations que dans la formule (III), et

R′, c et d ont les mêmes significations que dans la formule (IV).

Les produits de formules (V) et (VI) sont bien connus dans l'industrie des silicones et sont généralement disponibles dans le commerce.

Sur les produits de formules (V) et (VI), on fait réagir un alcool à insaturation alcénique de formule $R'_1OH$ où $R'_1$ est un radical alcénylène linéaire ou ramifié en $C_2$-$C_{18}$.

Parmi ces alcools, on utilise plus particulièrement l'alcool allylique ou l'alcool méthallylique.

On peut utiliser comme catalyseur d'hydrosilylation pour faire réagir les alcools insaturés sur l'hydrogènopolysiloxane de formule (V) ou (VI), des catalyseurs d'hydrosilylation connus, notamment les complexes du platine décrits dans les brevets US-A-3.715.334, 3.775.452 et 3.814.730; les complexes platine-oléfine décrits dans les brevets US-A-3.159.601 et 3.159.662.

Le milieu cosmétiquement acceptable, conforme à la présente invention, est un milieu aqueux contenant au moins un agent moussant.

L'agent moussant, conforme à la présente invention, est choisi parmi les tensio-actifs anioniques synthétiques ou les savons constitués d'acides gras en $C_6$-$C_{20}$, neutralisés par un agent alcalin, tel que la triéthanolamine, l'hydroxyde de potassium, l'hydroxyde de sodium ou leurs mélanges.

Les tensio-actifs anioniques synthétiques sont choisis parmi les sels de métaux alcalins ou d'ammonium de dérivés à chaînes grasses (en $C_{14}$-$C_{22}$) des acides glutamique, iséthionique, sulfosuccinique, sulfoacétique, de la sarcosine ou de la taurine et plus particulièrement parmi les N-acylglutamates, les alkyl-iséthionates, les alkylsulfosuccinates, les acylamino-polyéthoxysulfosuccinates, les alkylsulfoacétates, les N-acylsarcosinates, les N-méthyl N-acyltaurates, les radicaux alkyle et acyle comportant de 14 à 22 atomes de carbone.

Les acides gras utilisés conformément à la présente invention, sont de préférence constitués de mélanges d'acides gras en $C_{16}$-$C_{20}$ et d'acides gras en $C_6$-$C_{20}$, et notamment des mélanges d'acide stéarique et d'acides gras de coprah ou d'acide stéarique et d'acide myristique ou encore d'acide stéarique et d'acide laurique.

La proportion en savon d'acides gras en $C_{16}$-$C_{20}$ est comprise entre 40 et 90% et de préférence entre 75 et 90% en poids par rapport au poids total de la quantité de savon; celle en savon d'acide gras en $C_6$-$C_{20}$ est comprise entre 10 et 60% et de préférence entre 10 et 25% en poids par rapport au poids total de la quantité de savon.

D'autres agents moussants peuvent être utilisés en association avec les savons d'acides gras ou les tensio-actifs anioniques moussants définis ci-dessus, comme par exemple des agents tensio-actifs non ioniques, amphotères ou anioniques différents de ceux cités précédemment, des polymères comme l'alcool polyvinylique.

Les compositions préférées conformes à l'invention, renferment de 10 à 85% en poids d'eau, de 0,5 à 80% en poids de savon constitué d'acides gras en $C_6$-$C_{20}$ et neutralisés par un agent alcalin ou de tensio-actif anionique tel que défini ci-dessus.

Les compositions de rasage de la peau conformes à l'invention, contiennent dans le milieu cosmétiquement acceptable, défini ci-dessus, un polyorganosiloxane à fonction acyloxyalkyle de formule (I) ou (II) dans des concentrations comprises entre 0,2 et 3% en poids et de préférence entre 0,5 et 2% en poids par rapport au poids total de la composition.

Ces compositions peuvent se présenter sous forme de crèmes, de gels, de gels automoussants, de pains solides et plus particulièrement de mousses aérosol.

Les compositions de rasage conformes à l'invention peuvent être conditionnées en aérosol pour être distribuées sous forme de mousses ou de gels automoussants.

On utilise dans ce cas la composition en présence d'un gaz propulseur choisi parmi les hydrocarbures volatils, tels que le n-butane, l'isobutane, le propane, dont on préfère plus particulièrement un mélange ternaire de n-butane, isobutane, propane, vendu par exemple par la Société ELF AQUITAINE sous la dénomination AEROGAZ 3,2 N ou des hydrocarbures partiellement ou totalement fluorés dont plus particulièrement le monofluorotrichlorométhane, le dichlorodifluorométhane (F12), le dichloro-1,2 tétrafluoro-1,1,2,2-éthane (F114), utilisés seuls ou en combinaison; des hydrocarbures chlorés et/ou fluorés de ce type sont vendus sous la dénomination de Fréon ou Dymel par la Société DU PONT DE NEMOURS. On peut également utiliser comme propulseur des mélanges de ces hydrocarbures volatils avec des hydrocarbures chlorés et/ou fluorés, comme par exemple un mélange de n-butane, isobutane, propane et de monofluorotrichlorométhane, ou des propulseurs comme le protoxyde d'azote, le gaz carbonique ou l'éther diméthylique.

Les compositions de rasage plus particulièrement préférées, selon l'invention, sont représentées par des mousses aérosol qui renferment de 75 à 85% en poids d'eau, de 0,5 à 15% en poids de savon ou de tensio-actif anionique défini ci-dessus, de 0,2 à 3% en poids d'un polyorganosiloxane de formule (I) ou (II), de 2 à 15% et plus particulièrement de 3 à 10% en poids d'un agent propulseur.

Les compositions conformes à l'invention peuvent contenir en plus du polyorganosiloxane de formule (I) ou (II), des adjuvants habituellement utilisés dans le domaine des compositions à raser, tels que des agents hydratants choisis parmi le sorbitol et la glycérine; des agents de transparence jouant également le rôle de solvant, tels que des glycols comme le propylèneglycol ou l'éthylèneglycol, des agents filmogènes, des agents adoucissants, comme par exemple des dérivés de lanoline ou des polymères cationiques ou des polyéthylèneglycols, des agents de traitement de la peau, tels que des agents cicatrisants, des huiles minérales, des alcools gras, des polymères, des épaississants, des agents stabilisants, comme par exemple des éthanolamides, des agents apaisants, tels que l'allantoïne, le camphre, le menthol, des agents tensio-actifs anioniques autres que les savons et ceux mentionnés ci-dessus, des agents tensio-actifs non-ioniques ou amphotères ou leurs mélanges, des colorants, des conservateurs, des antioxydants et des parfums.

Le procédé de rasage de la peau, conforme à la présente invention, consiste essentiellement à appliquer sur la peau une composition telle que définie ci-dessus, à raser celle-ci au moyen d'un rasoir mécanique et à faire suivre d'un rinçage à l'eau.

Les exemples suivants sont destinés à mieux illustrer l'invention sans toutefois la limiter.

EXEMPLES DE PREPARATION

EXEMPLE A

Préparation d'un composé de formule moyenne (I), dans laquelle :

$R = R' = CH_3$ , $R_1 = -(CH_2)_3-$

$p = 7,9$

$q = 1,4$

$r = 9,3$

$R''$ = mélange de radicaux $C_{16}H_{33}$ et $C_{18}H_{37}$.

Ce composé comporte en moyenne environ deux motifs

$$CH_3-\underset{|}{Si}-OH \quad O_{2/2}$$

Dans un ballon tricol de 3 litres, équipé d'un agitateur, muni d'un dispositif de chauffage, d'un réfrigérant ascendant et balayé par un courant d'azote par bullage dans le milieu réactionnel, on charge 800 g d'une huile polydiméthylsiloxane à fonction γ-hydroxypropylée (obtenue par réaction d'hydrosilylation d'alcool allylique par une huile polydiméthylpolyméthylhydrogénosiloxane en présence d'un catalyseur au platine) de structure moléculaire moyenne, déterminée par analyse RMN$^{29}$Si; répondant à la formule moyenne (III), dans laquelle :

$R_2 = R' = CH_3$

$R_1 = -(CH_2)_3-$

$a = 6,8$

$b = 6,8$

et comportant en moyenne un motif

$$CH_3-\underset{|}{Si}-OH \quad O_{2/2}$$

titrant 459,5 meq/100 g en fonction alcool propylique, 1207 g d'une coupe d'esters gras $R''CO_2Me$ ($R''$ ayant la composition :

| | |
|---|---|
| $C_{14}H_{29}$ | 0,5 |
| $C_{16}H_{33}$ | 37,0 |
| $C_{18}H_{37}$ | 61,0 |
| $C_{20}H_{41}$ | 1,5 |
| | 100% poids |

Le milieu est porté par chauffage à 120°C. Lorsque la température est atteinte, on ajoute 6,02 g d'acide paratoluènesulfonique monohydraté ($CH_3C_6H_4SO_3H$, $H_2O$).

On effectue la réaction sous agitation pendant 17 heures. En fin de réaction, on ajoute dans le milieu réactionnel 500 ml d'hexane, l'élimination du catalyseur acide est alors effectuée par lavage, neutralisation avec $NaHCO_3$ aqueux.

La phase hexanique est séchée sur $Na_2SO_4$ et filtrée. 1822,6 g d'une huile limpide de couleur ambrée où 85% des fonctions alcool sont transformées en fonction ester gras, sont obtenus après élimination de l'hexane par distillation à 110°C sous 1,33 KPa pendant 3 heures. L'huile prend l'aspect d'une cire lors du refroidissement à température ambiante.

L'analyse RMN$^{29}$Si indique la structure mentionnée ci-dessus.

La teneur résiduelle en fonction alcool propylique est de 37 meq/100 g et en esters gras méthyliques $C_{16}+C_{18}$ de 20% en poids.

EXEMPLE B

On effectue les mêmes opérations qu'à l'exemple A, sauf que l'on charge 250 g de l'huile γ-hydroxypropylée précédemment utilisée, 370,5 g de la coupe d'esters gras. Lorsque le milieu est homogène après chauffage à 110°C, on charge 12,41 g d'une terre acide KATALYSATOR KSF/O de type Montmorillonite.

Après 17 heures de réaction, le milieu réactionnel est filtré à chaud (80°C), 545,8 g d'huile sont obtenus, où 85% des fonctions alcool sont transformées en fonction ester gras.

La structure moléculaire déterminée par analyse RMN$^{29}$Si correspond à la formule moyenne (I) dans laquelle :

$R = R' = CH_3$ ; $R_1 = (CH_2)_3$-

p = 14,8

q = 2,6

r = 17,4

R″ = mélange de radicaux $C_{16}H_{33}$ et $C_{18}H_{37}$.

Ce composé comporte, en moyenne, environ trois motifs

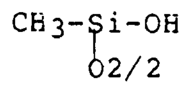

La teneur résiduelle en esters gras méthyliques $C_{16}$+$C_{18}$ est de 18,7% en poids.

EXEMPLES DE COMPOSITIONS

EXEMPLE 1

On prépare une mousse à raser aérosol de composition suivante :

| | | |
|---|---|---|
| - Acide stéarique | | 8,0 g |
| - Acide gras de coprah | | 1,0 g |
| - Hydroxyde de sodium | | 0,5 g |
| - Triéthanolamine | | 3,0 g |
| - Diorganopolysiloxane de l'exemple A | | 1,5 g |
| - Glycérine | | 2,5 g |
| - Tensio-actif amphotère dénommé ("Cocoamphocarboxyglycinate" dans le CTFA, 3ème édition, 1982), vendu sous le nom MIRANOL $C_2M$ conc., par la Société MIRANOL, en solution aqueuse à 38% de matière active | | 0,1 g MA |
| - Polyéthylèneglycol vendu par la Société UNION CARBIDE sous la dénomination Polyox coagulant grade | | 0,05 g |
| - Monolaurate de sorbitan polyoxyéthyléné à 20 moles d'oxyde d'éthylène, vendu sous la dénomination TWEEN 20 par la Société ATLAS | | 0,3 g |
| - Parfum qs | | |
| - Eau | qsp | 100,0 g |

Conditionnement aérosol :

| | | |
|---|---|---|
| Composition ci-dessus | | 97,0 g |
| Propulseur : Mélange ternaire de n-butane isobutane > 55%, propane vendu sous la dénomination AEROGAZ 3,2 N par la Société ELF AQUITAINE | | 3,0 g |
| | Total | 100,0 g |

EXEMPLE 2

On prépare une mousse à raser aérosol de composition suivante :

| | | |
|---|---|---|
| - Acide stéarique | | 5,5 g |
| - Acide myristique | | 1,2 g |
| - Hydroxyde de potassium | | 0,4 g |
| - Triéthanolamine | | 3,3 g |
| - Glycérine | | 5,0 g |
| - Acide lanolique | | 0,5 g |
| - Polyvinylpyrrolidone K 30 vendu par la Société GAF | | 1,0 g |
| - Diorganopolysiloxane de l'exemple A | | 1,0 g |
| - Myristate d'un mélange d'isopropanol-amines vendu sous la dénomination LANAMINE par la Société AMERCHOL | | 2,0 g |
| - Monolaurate de sorbitan polyoxy-éthyléné à 20 moles d'oxyde d'éthylène, vendu sous la dénomination TWEEN 20 par la Société ATLAS | | 0,5 g |
| - Parfum qs | | |
| - Eau | qsp | 100,0 g |

<u>Conditionnement aérosol</u> :

| | |
|---|---|
| Composition ci-dessus | 90,0 g |
| Propulseur : Fréons 12/114 (53/47) | 10,0 g |

<u>EXEMPLE 3</u>

On prépare une mousse à raser aérosol de composition suivante :

| | | | |
|---|---|---|---|
| - Acide stéarique | | 6,0 | g |
| - Acide gras de coprah | | 0,75 | g |
| - Hydroxyde de sodium | | 0,45 | g |
| - Triéthanolamine | | 2,25 | g |
| - Diorganopolysiloxane de l'exemple A | | 2,0 | g |
| - Sorbitol | | 5,0 | g |
| - Propylèneglycol | | 2,5 | g |
| - Copolymère de diméthyldiallylammonium et d'acrylamide vendu en solution aqueuse à 8% de matière active (MA) sous la dénomination MERQUAT S par la Société MERCK | | 0,5 | g MA |
| - Parfum qs | | | |
| - Eau | qsp | 100,0 | g |

Conditionnement aérosol :

| | | | |
|---|---|---|---|
| Composition ci-dessus | | 95,0 | g |
| Mélange ternaire de n-butane isobutane > 55% propane, vendu sous la dénomination AEROGAZ 3,2 N par la Société ELF AQUITAINE | | 5,0 | g |
| | Total | 100,0 | g |

EXEMPLE 4

On prépare un gel de rasage auto-moussant de composition suivante :

| | | | |
|---|---|---|---|
| - Acide stéarique | | 5,0 | g |
| - Acide palmitique | | 5,0 | g |
| - Triéthanolamine | | 5,55 | g |
| - Polyéthylèneglycoléther (20 OE) d'alcool oléique vendu sous la dénomination BRIJ 98 par la Société ICI | | 2,0 | g |
| - Hydroxyéthylcellulose vendue par la Société UNION CARBIDE sous la dénomination CELLOSIZE PCG 10 | | 1,35 | g |
| - Sorbitol | | 2,0 | g |
| - Diorganopolysiloxane de l'exemple A | | 2,0 | g |
| - Polyéthylèneglycol vendu sous la dénomination POLYOX WSR 205 par la Société UNION CARBIDE | | 0,5 | g |
| - Parfum, colorant qs | | | |
| - Eau | qsp | 100,0 | g |

On introduit 96 g de cette composition avec 2,5 g d'isopentane et 1,5 g d'isobutane dans la partie centrale d'un aérosol à double enveloppe, dont la paroi interne est constituée d'une membrane compressible, imperméable, séparant le propulseur (enveloppe extérieure) de la composition gélifiée auto-moussante (partie centrale).

Après sertissage de la valve, on pressurise la bombe aérosol en introduisant dans la double enveloppe 10% d'un mélange ternaire de n-butane-isobutane > 55% propane vendu sous la dénomination AEROGAZ 3,2 N par la Société ELF AQUITAINE.

EXEMPLE 5

On prépare une mousse à raser aérosol de composition suivante :

| | | |
|---|---|---|
| - Acide stéarique | | 8,3 g |
| - Acide gras de coprah | | 0,7 g |
| - Hydroxyde de potassium | | 0,9 g |
| - Triéthanolamine | | 3,1 g |
| - Glycérine | | 2,5 g |
| - Monolaurate de sorbitan polyoxyéthyléné à 20 moles d'oxyde d'éthylène, vendu sous la dénomination TWEEN 20 par la Société ATLAS | | 1,0 g |
| - Myristate d'un mélange d'isopropanol-amines vendu sous la dénomination LANAMINE par la Société AMERCHOL | | 2,0 g |
| - Diorganopolysiloxane de l'exemple B | | 2,0 g |
| - Parfum qs | | |
| - Eau | qsp | 100,0 g |

Conditionnement aérosol :

| | | |
|---|---|---|
| Composition ci-dessus | | 96,7 g |
| Propulseur : | | |
| Mélange ternaire de n-butane isobutane > 55% propane vendu sous la dénomination AEROGAZ 3,2 N par la Société ELF AQUITAINE | | 3,3 g |
| | Total | 100,0 g |

EXEMPLE 6

On prépare une mousse à raser de composition suivante :

| | | |
|---|---|---|
| - Sel monosodique du glutamate de formule : $HOOC - CH_2 - CH_2 - CH(NH - COR) - COONa$ où R est un mélange de radicaux alkényle et/ou alkyle hydrogénés en $C_{14}-C_{22}$ dérivés des acides gras du suif, vendu sous la dénomination ACYLGLUTAMATE HS 11 par la Société AJINOMOTO | | 3,0 g |
| - Sel disodique du glutamate de formule ci-dessus, vendu sous la dénomination ACYLGLUTAMATE HS 21 par la Société AJINOMOTO | | 3,5 g |
| - Glycérine | | 4,0 g |
| - Acide lanolique | | 1,0 g |
| - Tensio-actif amphotère dénommé "Cocoamphocarboxyglycinate"(CTFA, 3ème édition, 1982), vendu sous le nom de MIRANOL C2M Conc., par la Société MIRANOL, en solution aqueuse à 38% de matière active MA | | 1,0 g MA |
| - Hydroxyéthylcellulose vendue sous la dénomination CELLOSIZE PCG 10 par la Société UNION CARBIDE | | 0,2 g |
| - Polyéthylèneglycol vendu sous la dénomination POLYOX WSR 205 par la Société UNION CARBIDE | | 0,5 g |
| - Diorganopolysiloxane de l'exemple A | | 1,0 g |
| - Parfum qs | | |
| - Eau | qsp | 100,0 g |

Conditionnement aérosol :

| | |
|---|---|
| Composition ci-dessus | 96,0 g |
| Propulseur : | |
| Mélange ternaire de n-butane isobutane > 55% propane vendu sous la dénomination AEROGAZ 3,2 N par la Société ELF AQUITAINE | 4,0 g |
| Total | 100,0 g |

EXEMPLE 7

On prépare une mousse à raser de composition suivante :

| | | |
|---|---|---|
| - Oléamido-polyéthylèneglycol 2-sulfosuccinate disodique vendu sous la dénomination STANDAPOL SH 100 par la Société HENKEL à la concentration de 30% en matière active | | 7,5 g MA |
| - Sorbitol à 70% MA | | 5,0 g MA |
| - Hydroxyéthylcellulose vendue sous la dénomination CELLOSIZE PCG 10 par la Société UNION CARBIDE | | 0,8 g |
| - Diorganopolysiloxane de l'exemple A | | 1,5 g |
| - Parfum qs | | |
| - Eau | qsp | 100,0 g |

Conditionnement aérosol :

| | |
|---|---|
| Composition ci-dessus | 95,0 g |
| Propulseur : isobutane | 5,0 g |
| Total | 100,0 g |

## Revendications

### Revendications pour les Etats contractants suivants : AT, BE, CH, DE, FR, GB, GR, IT, LI, NL, SE

1. Composition destinée au rasage de la peau, contenant dans un milieux aqueux cosmétiquement acceptable, renfermant un agent moussant, un polyorganosiloxane à fonction acyloxyalkyle, choisi parmi :
   (i) les composés linéaires répondant à la formule moyenne (I) suivante :

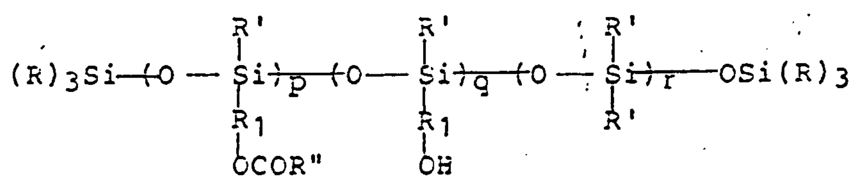

(I)

   dans laquelle :
   - les radicaux R, identiques ou différents, sont choisis parmi les radicaux méthyle, phényle, OCOR″, hydroxyle, un seul des R par atome de silicium peut être OH ;
   - les radicaux R′, identiques ou différents, sont choisis parmi les radicaux méthyle et phényle, 60% molaire au moins de l'ensemble des radicaux R et R′ est méthyle ;
   - $R_1$ représente un chaînon alkylène divalent hydrocarboné linéaire ou ramifié, comportant de 2 à 18 atomes de carbone ;
   - R″ est un radical alcoyle ou alcényle en $C_8$-$C_{20}$ ;
   - r est un nombre compris entre 1 et 120 inclus ;
   - p est un nombre compris entre 1 et 30 ;
   - q est égal à 0 ou est un nombre inférieur ou égal à 0,5 p, la somme p+q étant comprise entre 1 et 30 ;
   les composés de formule (I) pouvant éventuellement comporter des groupements

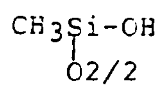

   présents dans des proportions ne dépassant pas 15% de la somme p+q+r ;
   (ii) les composés cycliques représentés par la formule moyenne (II) suivante :

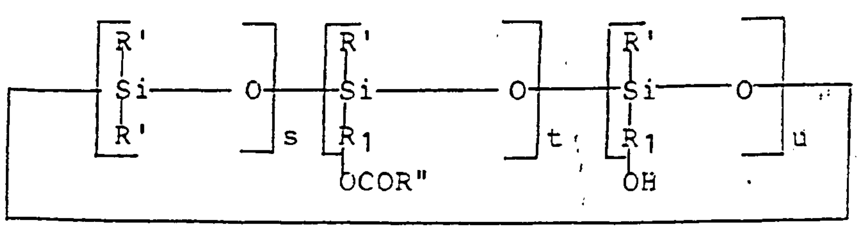

(II)

   dans laquelle :
   - R′, R″, $R_1$ ont la même signification que dans la formule (I) ;
   - s est un nombre compris entre 0 et 20 ;
   - t est un nombre compris entre 1 et 20 ;
   - u est égal à 0 ou est égal à un nombre inférieur ou égal à 0,5 t, la somme t+u étant comprise entre 1 et 20 ;
   la somme s+t+u étant supérieure ou égale à 3.

2. Composition selon la revendication 1, caractérisée par le fait que dans les composés de formule (I) ou (II), $R_1$ représente un chaînon alkylène divalent hydrocarboné, linéaire ou ramifié, comportant de 2 à 6 atomes de carbone.

3. Composition selon l'unes des revendications 1 à 2, caractérisée par le fait que dans les composés de formule (I) ou (II), R représente un radical méthyle, R′ représente un radical méthyle, $R_1$ représente un radical triméthylène, R″ désigne les groupements $C_{12}H_{25}$, $C_{14}H_{29}$, $C_{16}H_{33}$, $C_{18}H_{37}$, oléyle, r est compris entre 1 et 30, p est compris entre 5 et 16, q est compris entre 0,5 et 4, s+t+u est compris entre 3 et 10 inclus.

**4.** Composition selon l'une quelconque des revendications 1 à 3, caractérisée par le fait qu'elle contient dans un milieu cosmétiquement acceptable, renfermant un agent moussant, un polyorganosiloxane de formule (I) ou (II), dans des proportions comprises entre 0,2 et 3% en poids et de préférence entre 0,5 et 2% en poids par rapport au poids total de la composition.

**5.** Composition selon l'une quelconque des revendications 1 à 4, caractérisée par le fait qu'elle se présente sous forme de crèmes, de gels, de gels auto-moussants, de pains solides et de mousses aérosol.

**6.** Composition selon l'une quelconque des revendications 1 à 5, caractérisée par le fait que le milieu cosmétiquement acceptable est constitué par de l'eau et que l'agent moussant est un savon constitué par un acide gras en $C_6$-$C_{20}$ neutralisé par un agent alcalin ou un tensio-actif anionique synthétique.

**7.** Composition selon la revendication 6, caractérisée par le fait que l'agent moussant est un savon constitué d'un mélange d'acides gras en $C_6$-$C_{20}$ et d'acides gras en $C_{16}$-$C_{20}$, que la proportion en acides gras en $C_6$-$C_{20}$ est comprise entre 10 et 60% et de préférence entre 10 et 25% en poids par rapport au poids total de la quantité de savon et que la proportion en acides gras en $C_{16}$-$C_{20}$ est comprise entre 40 et 90% et de préférence entre 75 et 90% en poids par rapport au poids total de la quantité en savon.

**8.** Composition selon la revendication 6, caractérisée par le fait que l'agent tensio-actif anionique est choisi parmi les sels de métaux alcalins ou d'ammonium de dérivés à chaînes grasses (en $C_{14}$-$C_{22}$) des acides glutamique, iséthionique, sulfosuccinique, sulfoacétique, de la sarcosine ou de la taurine.

**9.** Composition selon l'une quelconque des revendications 1 à 8, caractérisée par le fait qu'elle renferme de 10 à 85% en poids d'eau et de 0,5 à 80% en poids d'agent moussant, tel que défini selon les revendications 6 à 8.

**10.** Composition selon l'une quelconque des revendications 1 à 9, caractérisée par le fait qu'elle contient un polymère moussant ou un agent tensio-actif moussant non-ionique, amphotère ou anionique différent des savons et de ceux définis dans la revendication 8.

**11.** Composition selon l'une quelconque des revendications 1 à 10, caractérisée par le fait qu'elle est conditionnée en aérosol en présence d'un gaz propulseur pour former au moment de l'expulsion un gel automoussant ou une mousse.

**12.** Composition selon la revendication 11, caractérisée par le fait qu'elle se présente sous forme de mousse aérosol, qu'elle renferme de 75 à 85% en poids d'eau, 0,5 à 15% en poids d'agent moussant tel que défini dans les revendications 6 à 8, 0,2 à 3% en poids d'un organopolysiloxane de formule (I) ou (II) et de 2 à 15% en poids d'un gaz propulseur et de préférence 3 à 10% en poids par rapport au poids total de la composition.

**13.** Composition selon l'une quelconque des revendications 1 à 12, caractérisée par le fait qu'elle contient en plus des agents hydratants, des agents de transparence, des agents filmogènes, des agents adoucissants, des agents cicatrisants, des huiles minérales, des alcools gras, des polymères, des épaississants, des agents stabilisants, des agents apaisants, des agents tensio-actifs anioniques autres que des savons et que ceux définis dans la revendication 8, non-ioniques ou amphotères ou leurs mélanges, des colorants, des conservateurs, des antioxydants et des parfums.

**14.** Procédé de rasage de la peau, caractérisé par le fait que l'on applique sur la peau une composition telle que définie dans l'une quelconque des revendications 1 à 13, que l'on rase la peau au moyen d'un rasoir mécanique et que l'on rince à l'eau.

**Revendications pour l'Etat contractant suivant : ES**

**1.** Procédé de préparation d'une composition destinée au rasage de la peau, incorporant dans un milieu aqueux cosmétiquement acceptable, renfermant un agent moussant, un polyorganosiloxane à fonction

acyloxyalkyle, choisi parmi :

(i) les composés linéaires répondant à la formule moyenne (I) suivante :

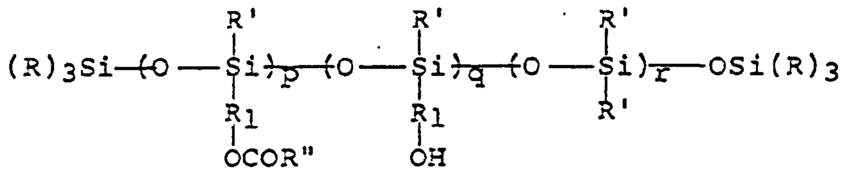

(I)

dans laquelle :

- les radicaux R, identiques ou différents, sont choisi parmi les radicaux méthyle, phényle, OCOR″, hydroxyle, un seul des R par atome de silicium peut être OH ;
- les radicaux R′, identiques ou différents, sont choisis parmi les radicaux méthyle et phényle, 60% molaire au moins de l'ensemble des radicaux R et R′ est méthyle ;
- $R_1$ représente un chaînon alkylène divalent hydrocarboné, linéaire ou ramifié, comportant de 2 à 18 atomes de carbone ;
- R″ est un radical alcoyle ou alcényle en $C_8$-$C_{20}$ ;
- r est un nombre compris entre 1 et 120 inclus ;
- p est un nombre compris entre 1 et 30 ;
- q est égal à 0 ou est un nombre inférieur ou égal à 0,5 p, la somme p+q étant comprise entre 1 et 30 ;

les composés de formule (I) pouvant éventuellement comporter des groupements

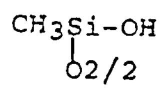

présents dans des proportions ne dépassant pas 15% de la somme p+q+r ;

(ii) les composés cycliques représentés par la formule moyenne (II) suivante :

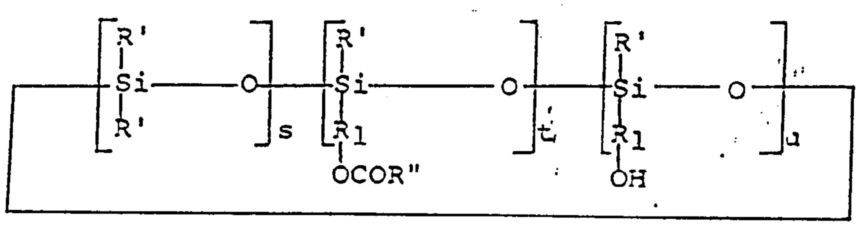

(II)

dans laquelle :

- R′, R″, $R_1$ ont la même signification que dans la formule (I) ;
- s est un nombre compris entre 0 et 20 ;
- t est un nombre compris entre 1 et 20 ;
- u est égal à 0 ou est égal à un nombre inférieur ou égal à 0,5 t, la somme t+u étant comprise entre 1 et 20 ;

la somme s+t+u étant supérieure ou égale à 3.

**2.** Composition destinée au rasage de la peau, contenant dans un milieu aqueux cosmétiquement acceptable, renfermant un agent moussant, un polyorganosiloxane à fonction acyloxyalkyle, choisi parmi :

(i) les composés linéaires répondant à la formule moyenne (I) suivante :

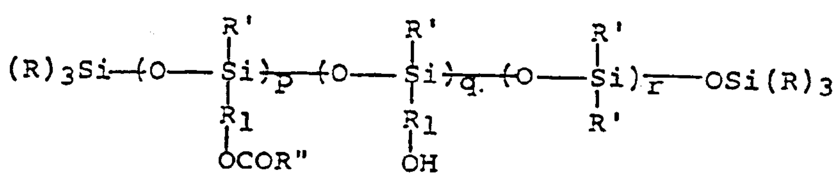

(I)

dans laquelle :

- les radicaux R, identiques ou différents, sont choisis parmi les radicaux méthyle, phényle, OCOR″, hydroxyle, un seul des R par atome de silicium peut être OH ;
- les radicaux R′, identiques ou différents, sont choisis parmi les radicaux méthyle et phényle, 60% molaire au moins de l'ensemble des radicaux R et R′ est méthyle ;
- $R_1$ représente un chaînon alkylène divalent hydrocarboné, linéaire ou ramifié, comportant de 2 à 18 atomes de carbone ;
- R″ est un radical alcoyle ou alcényle en $C_8$-$C_{20}$ ;
- r est un nombre compris entre 1 et 120 inclus ;
- p est un nombre compris entre 1 et 30 ;
- q est égal à 0 ou est un nombre inférieur ou égal à 0,5 p, la somme p+q étant comprise entre 1 et 30 ;

les composés de formule (I) pouvant éventuellement comporter des groupements

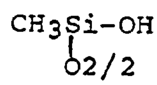

présents dans des proportions ne dépassant pas 15% de la somme p+q+r ;
(ii) les composés cycliques représentés par la formule moyenne (II) suivante :

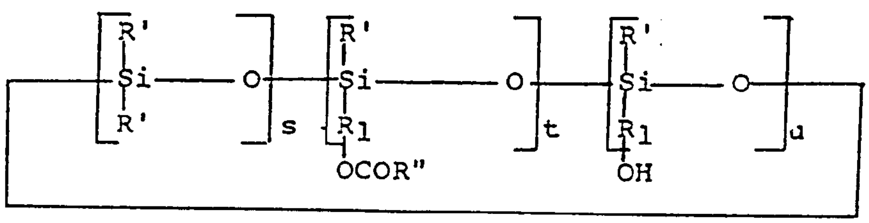

(II)

dans laquelle :

- R′, R″, $R_1$ ont la même signification que dans la formule (I) :
- s est un nombre compris entre 0 et 20 ;
- t est un nombre compris entre 1 et 20 ;
- u est égal à 0 ou est égal à un nombre inférieur ou égal à 0,5 t, la somme t+u étant comprise entre 1 et 20;

la somme s+t+u étant supérieure ou égale à 3.

3. Composition selon la revendication 2, caractérisée par le fait que dans les composés de formule (I) ou (II), $R_1$ représente un chaînon alkylène divalent hydrocarboné, linéaire ou ramifié, comportant de 2 à 6 atomes de carbone.

4. Composition selon l'une des revendications 2 à 3, caractérisée par le fait que dans les composés de formule (I) ou (II), R représente un radical méthyle, R′ représente un radical méthyle, $R_1$ représente un radical triméthylène, R″ désigne les groupements $C_{12}H_{25}$, $C_{14}H_{29}$, $C_{16}H_{33}$, $C_{18}H_{37}$, oléyle, r est compris entre 1 et 30, p est compris entre 5 et 16, q est compris entre 0,5 et 4, s+t+u est compris entre 3 et 10 inclus.

5. Composition selon l'une quelconque des revendications 2 à 4, caractérisée par le fait qu'elle contient dans un milieu cosmétiquement acceptable, renfermant un agent moussant, un polyorganosiloxane de formule (I) ou (II), dans des proportions comprises entre 0,2 et 3% en poids et de préférence entre 0,5 et 2% en poids par rapport au poids total de la composition.

6. Composition selon l'une quelconque des revendications 2 à 5, caractérisée par le fait qu'elle se présente sous forme de crèmes, de gels, de gels anti-moussants, de pains solides et de mousses aérosol.

7. Composition selon l'une quelconque des revendications 2 à 6, caractérisée par le fait que le milieu cosmétiquement acceptable est constitué par de l'eau et que l'agent moussant est un savon constitué par un acide gras en $C_6$-$C_{20}$ neutralisé par un agent alcalin ou un tensio-actif anionique synthétique.

8. Composition selon la revendication 7, caractérisée par le fait que l'agent moussant est un savon constitué d'un mélange d'acides gras en $C_6$-$C_{20}$ et d'acides gras en $C_{16}$-$C_{20}$, que la proportion en acides gras en

$C_6$-$C_{20}$ est comprise entre 10 et 60% et de préférence entre 10 et 25% en poids par rapport au poids total de la quantité de savon et que la proportion en acides gras en $C_{16}$-$C_{20}$ est comprise entre 40 et 90% et de préférence entre 75 et 90% en poids par rapport au poids total de la quantité en savon.

**9.** Composition selon la revendication 7, caractérisée par le fait que l'agent tensio-actif anionique est choisi parmi les sels de métaux alcalins ou d'ammonium de dérivés à chaînes grasses (en $C_{14}$-$C_{22}$) des acides glutamique, iséthionique, sulfosuccinique, sulfoacétique, de la sarcosine ou de la taurine.

**10.** Composition selon l'une quelconque des revendications 2 à 9, caractérisée par le fait qu'elle renferme de 10 à 85% en poids d'eau et de 0,5 à 80% en poids d'agent moussant, tel que défini selon les revendications 7 à 9.

**11.** Composition selon l'une quelconque des revendications 2 à 10, caractérisée par le fait qu'elle contient un polymère moussant ou un agent tensio-actif moussant non-ionique, amphotère ou anionique différent des savons et de ceux définis dans la revendication 8.

**12.** Composition selon l'une quelconque des revendications 2 à 11, caractérisée par le fait qu'elle est conditionnée en aérosol en présence d'un gaz propulseur pour former au moment de l'expulsion un gel automoussant ou une mousse.

**13.** Composition selon la revendication 12, caractérisée par le fait qu'elle se présente sous forme de mousse aérosol, qu'elle renferme de 75 à 85% en poids d'eau, 0,5 à 15% en poids d'agent moussant tel que défini dans les revendications 6 à 8, 0,2 à 3% en poids d'un organopolysiloxane de formule (I) ou (II) et de 2 à 15% en poids d'un gaz propulseur et de préférence 3 à 10% en poids par rapport au poids total de la composition.

**14.** Composition selon l'une quelconque des revendications 2 à 13, caractérisée par le fait qu'elle contient en plus des agents hydratants, des agents de transparence, des agents filmogènes, des agents adoucissants, des agents cicatrisants, des huiles minérales, des alcools gras, des polymères, des épaississants, des agents stabilisants, des agents apaisants, des agents tensio-actifs anioniques autres que des savons et que ceux définis dans la revendication 8, non-ioniques ou amphotères ou leurs mélanges, des colorants, des conservateurs, des antioxydants et des parfums.

**15.** Procédé de rasage de la peau, caractérisé par le fait que l'on applique sur la peau une composition telle que définie dans l'une quelconque des revendications 2 à 14, que l'on rase la peau au moyen d'un rasoir mécanique et que l'on rince à l'eau.

## Claims

### Claims for the following Contracting States: AT, BE, CH, DE, FR, GB, GR, IT, LI, NL, SE

**1.** Composition intended for shaving the skin, containing in a cosmetically acceptable aqueous medium, enclosing a foaming agent, a polyorganosiloxane containing an acyloxyalkyl functional group, chosen from:
(i) the linear compounds corresponding to the following average formula (I):

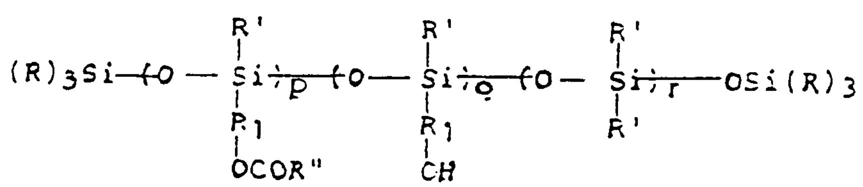 (I)

in which:

- the radicals R, which are identical or different, are chosen from methyl, phenyl, OCOR″ and hydroxyl radicals, only one of the R per silicon atom may be OH;
- the radicals R′, which are identical or different, are chosen from methyl and phenyl radicals, at least 60 mol% of the total of the radicals R and R′ is methyl;
- $R_1$ denotes a linear or branched divalent alkylene hydrocarbon chain unit containing from 2 to 18

carbon atoms;
- R″ is a $C_8$-$C_{20}$ alkyl or alkenyl radical;
- r is a number between 1 and 120 inclusive;
- p is a number between 1 and 30;
- q is equal to 0 or is a number smaller than or equal to 0.5 p, the sum p+q being between 1 and 30;

it being optionally possible for the compounds of formula (I) to contain groups

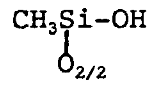

which are present in proportions not exceeding 15 % of the sum p+q+r;
(ii) the cyclic compounds denoted by the following average formula (II):

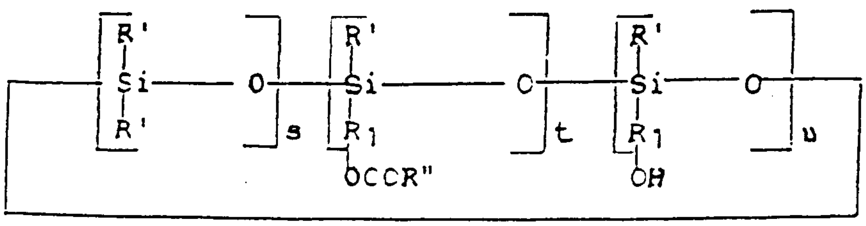 (II)

in which:
- R′, R″ and $R_1$ have the same meaning as in formula (I);
- s is a number between 0 and 20;
- t is a number between 1 and 20;
- u is equal to 0 or is equal to a number smaller than or equal to 0.5 t, the sum t+u being between 1 and 20;

the sum s+t+u being greater than or equal to 3.

**2.** Composition according to Claim 1, characterised in that in the compounds of formula (I) or (II) $R_1$ denotes a linear or branched divalent alkylene hydrocarbon chain unit containing from 2 to 6 carbon atoms.

**3.** Composition according to either of Claims 1 and 2, characterised in that in the compounds of formula (I) or (II) R denotes a methyl radical, R′ denotes a methyl radical, $R_1$ denotes a trimethylene radical, R″ denotes the groups $C_{12}H_{25}$, $C_{14}H_{29}$, $C_{16}H_{33}$, $C_{18}H_{37}$, and oleyl, r is between 1 and 30, p is between 5 and 16, q is between 0.5 and 4 and s+t+u is between 3 and 10 inclusive.

**4.** Composition according to any one of Claims 1 to 3, characterised in that it contains in a cosmetically acceptable medium, enclosing a foaming agent, a polyorganosiloxane of formula (I) or (II) in proportions of between 0.2 and 3 % by weight and preferably between 0.5 and 2 % by weight relative to the total weight of the composition.

**5.** Composition according to any one of Claims 1 to 4, characterised in that it is in the form of creams, gels, self-foaming gels, solid cakes and aerosol foams.

**6.** Composition according to any one of Claims 1 to 5, characterised in that the cosmetically acceptable medium consists of water and that the foaming agent is a soap consisting of a $C_6$-$C_{20}$ fatty acid neutralised by an alkaline agent or a synthetic anionic surfactant.

**7.** Composition according to Claim 6, characterised in that the foaming agent is a soap consisting of a mixture of $C_6$-$C_{20}$ fatty acids and of $C_{16}$-$C_{20}$ fatty acids, that the proportion of $C_6$-$C_{20}$ fatty acids is between 10 and 60 % and preferably between 10 and 25 % by weight relative to the total weight of the quantity of soap and that the proportion of $C_{16}$-$C_{20}$ fatty acids is between 40 and 90 % and preferably between 75 and 90 % by weight relative to the total weight of the quantity as soap.

**8.** Composition according to Claim 6, characterised in that the anionic surface-active agent is chosen from the alkali metal or ammonium salts of fatty chain ($C_{14}$-$C_{22}$) derivatives of glutamic, isethionic, sulphosuccinic and sulphoacetic acids, of sarcosine or of taurine.

**9.** Composition according to any one of Claims 1 to 8, characterised in that it encloses from 10 to 85 % by weight of water and from 0.5 to 80 % by weight of foaming agent as defined in Claims 6 to 8.

**10.** Composition according to any one of Claims 1 to 9, characterised in that it contains a foaming polymer or a nonionic, amphoteric or anionic foaming surface-active agent other than soaps and other than those defined in Claim 8.

**11.** Composition according to any one of Claims 1 to 10, characterised in that it is packaged as an aerosol in the presence of a propellent gas to form a self-foaming gel or a foam at the time of the expulsion.

**12.** Composition according to Claim 11, characterised in that it is in the form of aerosol foam, that it contains from 75 to 85 % by weight of water, 0.5 to 15 % by weight of foaming agent as defined in Claims 6 to 8, 0.2 to 3 % by weight of an organopolysiloxane of formula (I) or (II) and from 2 to 15 % by weight of a propellent gas and preferably 3 to 10 % by weight relative to the total weight of the composition.

**13.** Composition according to any one of Claims 1 to 12, characterised in that it additionally contains hydrating agents, transparency agents, film-forming agents, emollient agents, cicatrising agents, mineral oils, fatty alcohols, polymers, thickeners, stabilising agents, soothing agents, anionic surface-active agents other than soaps and than those defined in Claim 8, nonionic or amphoteric ones or mixtures thereof, colorants, preserving agents, antioxidants and perfumes.

**14.** Process for shaving the skin, characterised in that a composition as defined in any one of Claims 1 to 13 is applied to the skin, that the skin is shaved with a mechanical razor and that it is rinsed with water.

**Claims for the following Contracting State : ES**

**1.** Process for the preparation of a composition intended for shaving the skin, containing in a cosmetically acceptable aqueous medium, enclosing a foaming agent, a polyorganosiloxane containing an acyloxyalkyl functional group, chosen from:

(i) the linear compounds corresponding to the following average formula (I):

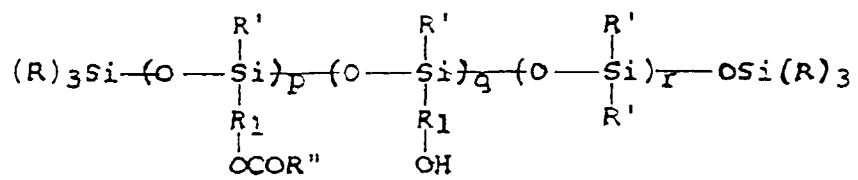 (I)

in which:

- the radicals R, which are identical or different, are chosen from methyl, phenyl, OCOR″ and hydroxyl radicals, only one of the R per silicon atom may be OH;
- the radicals R′, which are identical or different, are chosen from methyl and phenyl radicals, at least 60 mol% of the total of the radicals R and R′ is methyl;
- $R_1$ denotes a linear or branched divalent alkylene hydrocarbon chain unit containing from 2 to 18 carbon atoms;
- R″ is a $C_8$-$C_{20}$ alkyl or alkenyl radical;
- r is a number between 1 and 120 inclusive;
- p is a number between 1 and 30;
- q is equal to 0 or is a number smaller than or equal to 0.5 p, the sum p+q being between 1 and 30;

it being optionally possible for the compounds of formula (I) to contain groups

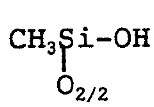

which are present in proportions not exceeding 15 % of the sum p+q+r;

(ii) the cyclic compounds denoted by the following average formula (II):

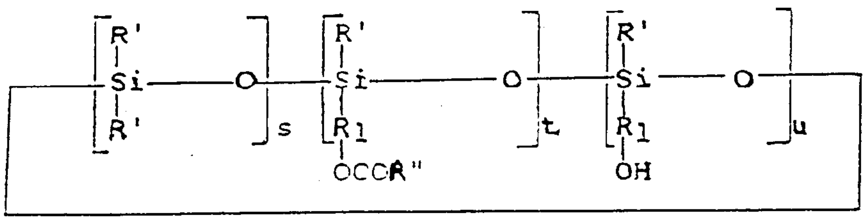

(II)

in which:

- R′, R″ and $R_1$ have the same meaning as in formula (I);
- s is a number between 0 and 20;
- t is a number between 1 and 20;
- u is equal to 0 or is equal to a number smaller than or equal to 0.5 t, the sum t+u being between 1 and 20;

the sum s+t+u being greater than or equal to 3.

**2.** Composition intended for shaving the skin, containing in a cosmetically acceptable aqueous medium, enclosing a foaming agent, a polyorganosiloxane containing an acyloxyalkyl functional group, chosen from:

(i) the linear compounds corresponding to the following average formula (I):

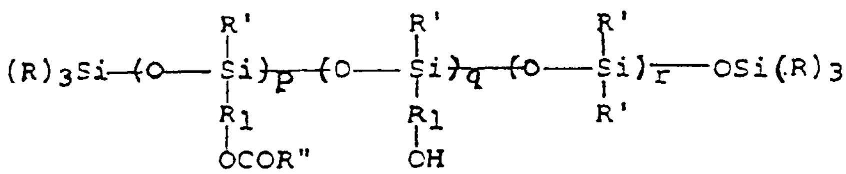

(I)

in which:

- the radicals R, which are identical or different, are chosen from methyl, phenyl, OCOR″ and hydroxyl radicals, only one of the R per silicon atom may be OH;
- the radicals R′, which are identical or different, are chosen from methyl and phenyl radicals, at least 60 mol% of the total of the radicals R and R′ is methyl;
- $R_1$ denotes a linear or branched divalent alkylene hydrocarbon chain unit containing from 2 to 18 carbon atoms;
- R″ is a $C_8$-$C_{20}$ alkyl or alkenyl radical;
- r is a number between 1 and 120 inclusive;
- p is a number between 1 and 30;
- q is equal to 0 or is a number smaller than or equal to 0.5 p, the sum p+q being between 1 and 30;

it being optionally possible for the compounds of formula (I) to contain groups

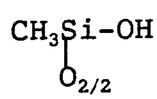

which are present in proportions not exceeding 15 % of the sum p+q+r;

(ii) the cyclic compounds denoted by the following average formula (II):

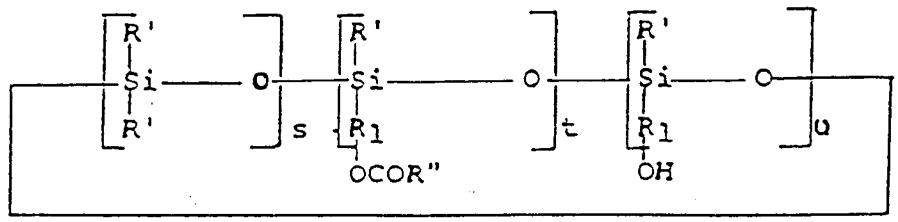

(II)

in which:

- R′, R″ and $R_1$ have the same meaning as in formula (I);
- s is a number between 0 and 20;
- t is a number between 1 and 20;
- u is equal to 0 or is equal to a number smaller than or equal to 0.5 t, the sum t+u being between 1 and 20;

the sum s+t+u being greater than or equal to 3.

**3.** Composition according to Claim 2, characterised in that in the compounds of formula (I) or (II) $R_1$ denotes a linear or branched divalent alkylene hydrocarbon chain unit containing from 2 to 6 carbon atoms.

**4.** Composition according to either of Claims 2 and 3, characterised in that in the compounds of formula (I) or (II) R denotes a methyl radical, R′ denotes a methyl radical, $R_1$ denotes a trimethylene radical, R″ denotes the groups $C_{12}H_{25}$, $C_{14}H_{29}$, $C_{16}H_{33}$, $C_{18}H_{37}$ and oleyl, r is between 1 and 30, p is between 5 and 16, q is between 0.5 and 4 and s+t+u is between 3 and 10 inclusive.

**5.** Composition according to any one of Claims 2 to 4, characterised in that it contains in a cosmetically acceptable medium, enclosing a foaming agent, a polyorganosiloxane of formula (I) or (II) in proportions of between 0.2 and 3 % by weight and preferably between 0.5 and 2 % by weight relative to the total weight of the composition.

**6.** Composition according to any one of Claims 2 to 5, characterised in that it is in the form of creams, gels, self-foaming gels, solid cakes and aerosol foams.

**7.** Composition according to any one of Claims 2 to 6, characterised in that the cosmetically acceptable medium consists of water and that the foaming agent is a soap consisting of a $C_6$-$C_{20}$ fatty acid neutralised by an alkaline agent or a synthetic anionic surfactant.

**8.** Composition according to Claim 7, characterised in that the foaming agent is a soap consisting of a mixture of $C_6$-$C_{20}$ fatty acids and of $C_{16}$-$C_{20}$ fatty acids, that the proportion of $C_6$-$C_{20}$ fatty acids is between 10 and 60 % and preferably between 10 and 25 % by weight relative to the total weight of the quantity of soap and that the proportion of $C_{16}$-$C_{20}$ fatty acids is between 40 and 90 % and preferably between 75 and 90 % by weight relative to the total weight of the quantity as soap.

**9.** Composition according to Claim 7, characterised in that the anionic surface-active agent is chosen-from the alkali metal or ammonium salts of fatty chain ($C_{14}$-$C_{22}$) derivatives of glutamic, isethionic, sulphosuccinic and sulphoacetic acids, of sarcosine or of taurine.

**10.** Composition according to any one of Claims 2 to 9, characterised in that it encloses from 10 to 85 % by weight of water and from 0.5 to 80 % by weight of foaming agent as defined in Claims 7 to 9.

**11.** Composition according to any one of Claims 2 to 10, characterised in that it contains a foaming polymer or a nonionic, amphoteric or anionic foaming surface-active agent other than soaps and other than those defined in Claim 8.

**12.** Composition according to any one of Claims 2 to 11, characterised in that it is packaged as an aerosol in the presence of a propellent gas to form a self-foaming gel or a foam at the time of the expulsion.

**13.** Composition according to Claim 12, characterised in that it is in the form of aerosol foam, that it contains from 75 to 85 % by weight of water, 0.5 to 15 % by weight of foaming agent as defined in Claims 6 to 8, 0.2 to 3 % by weight of an organopolysiloxane of formula (I) or (II) and from 2 to 15 % by weight of a propellent gas and preferably 3 to 10 % by weight relative to the total weight of the composition.

**14.** Composition according to any one of Claims 2 to 13, characterised in that it additionally contains hydrating agents, transparency agents, film-forming agents, emollient agents, cicatrising agents, mineral oils, fatty alcohols, polymers, thickeners, stabilising agents, soothing agents, anionic surface-active agents other than soaps and than those defined in Claim 8, nonionic or amphoteric ones or mixtures thereof, colorants, preserving agents, antioxidants and perfumes.

**15.** Process for shaving the skin, characterised in that a composition as defined in any one of Claims 2 to 14 is applied to the skin, that the skin is shaved with a mechanical razor and that it is rinsed with water.

## Patentansprüche

### Patentansprüche für folgende Vertragsstaaten : AT, BE, CH, DE, FR, GB, GR, IT, LI, NL, SE

**1.** Zusammensetzung zur Rasur der Haut, enthaltend in einem kosmetischen verträglichen wässrigen Medium, das ein Schaummittel enthält, ein Polyorganosiloxan mit Acyloxyalkylfunktion, ausgewählt aus:

(i) linearen Verbindungen gemäß der folgenden Durchschnittsformel (I):

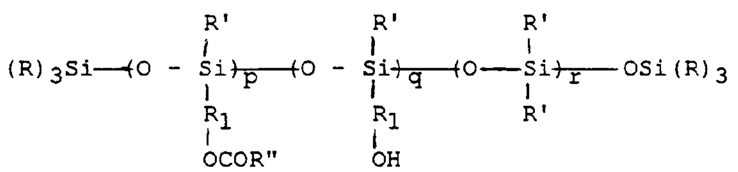 (I)

worin:

- die Reste R, gleich oder verschieden, unter Methyl-, Phenyl-, -OCOR″- und Hydroxylresten ausgewählt sind, wobei nur einer der Reste R pro Siliziumatom OH sein kann;
- die Reste R′, gleich oder verschieden, unter Methyl- und Phenylresten ausgewählt sind, wobei mindestens 60 Mol% aller Reste R und R′ Methylreste sind;
- $R_1$ eine zweiwertige lineare oder verzweigte Kohlenwasserstoff-Alkylenkette mit 2 bis 18 Kohlenstoffatomen, vorzugsweise 2 bis 6 Kohlenstoffatomen, ist, und insbesondere eine Trimethylenkette $-(CH_2)_3-$ und eine 2-Methyltrimethylenkette

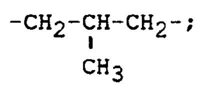

- R″ ein $C_{8-20}$-Alkyl- oder -Alkenylrest ist;
- r eine Zahl von 1 bis 120 ist;
- p eine Zahl von 1 bis 30 ist;
- q gleich Null oder eine Zahl unterhalb oder gleich 0,5 p ist, wobei die Summe p+q 1 bis 30 beträgt;
- wobei die Verbindung der Formel (I) gegebenenfalls Gruppierungen

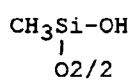

enthalten können, die in Mengen vorliegen, die 15 % der Summe p+q+r nicht übersteigen;

(ii) zyklischen Verbindungen gemäß der folgenden Durchschnittsformel (II):

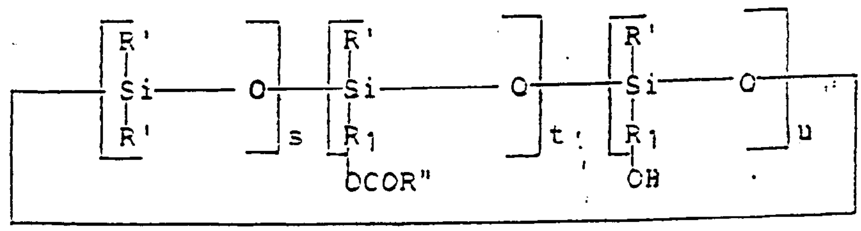

(II)

worin:

R′, R″ und $R_1$ die oben angegebenen Bedeutungen haben;

- s eine Zahl von 0 bis 20 ist;
- t eine Zahl von 1 bis 20 ist;
- u gleich Null oder gleich einer Zahl unterhalb oder gleich 0,5 t ist, wobei die Summe t+u 1 bis 20 beträgt, und

wobei die Summe s+t+u größer oder gleich 3 ist.

**2.** Zusammensetzung gemäß Anspruch 1, dadurch **gekennzeichnet**, daß in den Verbindungen der Formel (I) oder (II) $R_1$ eine lineare oder verzweigte zweiwertige Alkylenkohlenwasserstoffkette mit 2 bis 6 Kohlenstoffatomen darstellt.

**3.** Zusammensetzung gemäß einem der Ansprüche 1 bis 2, dadurch **gekennzeichnet**, daß in den Verbindungen der Formel (I) oder (II) R einen Methylrest, R′ einen Methylrest, $R_1$ einen Trimethylenrest darstellen, R″ die Gruppen $C_{12}H_{25}$, $C_{14}H_{29}$, $C_{16}H_{33}$ $C_{18}H_{37}$ und der Oleylrest bedeutet, r 1 bis 30, p 5 bis 16, q 0 bis 4 betragen und s+t+u 3 bis 10 beträgt.

**4.** Zusammensetzung gemäß jedem Anspruch 1 bis 3, dadurch **gekennzeichnet**, daß sie in einem kosmetisch verträglichen Medium, enthaltend ein Schaummittel, ein Polyorganosiloxan der Formel (I) oder (II) in Mengenverhältnissen von 0,2 bis 3 Gew.%, vorzugsweise 0,5 bis 2 Gew.%, bezogen auf Gesamtgewicht der Zusammensetzung, enthält.

**5.** Zusammensetzung gemäß jedem Anspruch 1 bis 4, dadurch **gekennzeichnet**, daß sie in Form von Cremes, Gelen, selbstschäumenden Gelen, festen Seifestücken und Aerosolschäumen vorliegt.

**6.** Zusammensetzung gemäß jedem Anspruch 1 bis 5, dadurch **gekennzeichnet**, daß das kosmetisch verträgliche Medium aus Wasser zusammengesetzt ist und daß das Schaummittel eine Seife aus einer $C_{6-20}$-Fettsäure, neutralisiert durch ein alkalisches Mittel, oder ein synthetisches anionisches oberflächenaktives Mittel ist.

**7.** Zusammensetzung gemäß Anspruch 6, dadurch **gekennzeichnet**, daß das Schaumittel eine Seife aus einer Mischung von $C_{6-20}$-Fettsäuren und $C_{16-20}$-Fettsäuren ist, daß das Mengenverhältnis an $C_{6-20}$-Fettsäuren 10 bis 60 Gew.%, vorzugsweise 10 bis 25 Gew.%, bezogen auf Gesamtmenge der Seife, und das Mengenverhältnis an $C_{16-20}$-Fettsäuren 40 bis 90 Gew.%, vorzugsweise 75 bis 90 Gew.%, bezogen auf Gesamtmenge an Seife, betragen.

**8.** Zusammensetzung gemäß Anspruch 6, dadurch **gekennzeichnet**, daß das anionische oberflächenaktive Mittel ausgewählt ist aus Alkalimetall- und Ammoniumsalzen von Derivaten mit $C_{14-22}$-Fettsäureketten von Glutamin-, Isethion-, Sulfobernstein- und Sulfoessigsäuren, aus Sarcosin oder Taurin.

**9.** Zusammensetzung gemäß jedem der Ansprüche 1 bis 8, dadurch **gekennzeichnet**, daß sie 10 bis 85 Gew.% Wasser und 0,5 bis 80 Gew.% eines gemäß der Ansprüche 6 bis 8 definierten Schaummittels enthält.

**10.** Zusammensetzung gemäß jedem der Ansprüche 1 bis 9, dadurch **gekennzeichnet**, daß sie ein schäumendes Polymer oder ein von den Seifen und den in Anspruch 8 definierten Mitteln unterschiedenes nichtionisches, amphoteres oder anionisches schäumendes oberflächenaktives Mittel enthält.

**11.** Zusammensetzung gemäß jedem Anspruch 1 bis 10, dadurch **gekennzeichnet**, daß sie als Aerosol in Gegenwart eines Treibmittelgases zubereitet ist, um in Augenblick der Freisetzung ein selbstschäumendes Gel oder einen Schaum zu bilden.

12. Zusammensetzung gemäß Anspruch 11, dadurch **gekennzeichnet**, daß sie in Form eines Aerosolschaumes vorliegt, der 75 bis 85 Gew.% Wasser, 0,5 bis 15 Gew.% eines in den Ansprüchen 6 bis 8 definierten Schaummittels, 0,2 bis 3 Gew.% eines Organopolysiloxans der Formel (I) oder (II) und 2 bis 15 Gew.%, vorzugsweise 3 bis 10 Gew.%, eines Treibmittelgases enthält, bezogen auf Gesamtgewicht der Zusammensetzung.

13. Zusammensetzung gemäß jedem der Ansprüche 1 bis 12, dadurch **gekennzeichnet**, daß sie zusätzlich Feuchtigkeitsmittel, Transparenzmittel, filmbildende Mittel, weichmachende Mittel, Vernarbungsmittel, Mineralöle, Fettalkohole, Polymere, Verdickungsmittel, Stabilisierungsmittel, Beruhigungsmittel, anionische oberflächenaktive Mittel, die sich von den Seifen und den in Anspruch 8 definierten Mitteln unterscheiden, nicht-ionische oder amphotere Mittel oder deren Mischungen, Färbemittel, Konservierungsmittel, Antioxidantien und Parfüms enthält.

14. Verfahren zum Rasieren der Haut, dadurch **gekennzeichnet**, daß man auf die Haut eine gemäß jedem der Ansprüche 1 bis 13 definierte Zusammensetzung aufträgt, die Haut mittels eines mechanischen Rasierers rasiert und mit Wasser spült.

**Patentansprüche für folgenden Vertragsstaat : ES**

1. Verfahren zur Herstellung einer Zusammensetzung zum Rasieren der Haut, wobei man in ein kosmetisch verträgliches wässriges Medium, enthaltend ein Schaummittel, ein Polyorganosiloxan mit Acyloxyalkylfunktion einbringt, ausgewählt aus:
(i) linearen Verbindungen gemäß der folgenden Durchschnittsformel (I):

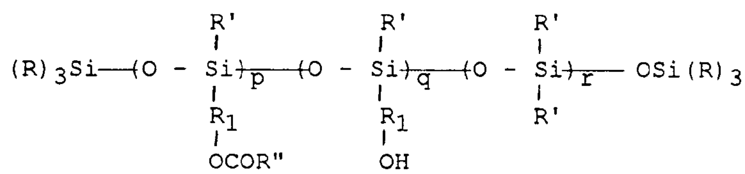 (I)

worin:
- die Reste R, gleich oder verschieden, unter Methyl-, Phenyl-, -OCOR″- und Hydroxylresten ausgewählt sind, wobei nur einer der Reste R pro Siliziumatom OH sein kann;
- die Reste R′, gleich oder verschieden, unter Methyl- und Phenylresten ausgewählt sind, wobei mindestens 60 Mol% aller Reste R und R′ Methylreste sind;
- $R_1$ eine zweiwertige lineare oder verzweigte Kohlenwasserstoff-Alkylenkette mit 2 bis 18 Kohlenstoffatomen, vorzugsweise 2 bis 6 Kohlenstoffatomen, ist, und insbesondere eine Trimethylenkette $-(CH_2)_3-$ und eine 2-Methyltrimethylenkette

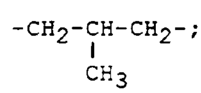

- R″ ein $C_{8-20}$-Alkyl- oder -Alkenylrest ist;
- r eine Zahl von 1 bis 120 ist;
- p eine Zahl von 1 bis 30 ist;
- q gleich Null oder eine Zahl unterhalb oder gleich 0,5 p ist, wobei die Summe p+q 1 bis 30 beträgt;
wobei die Verbindungen der Formel (I) gegebenenfalls Gruppierungen

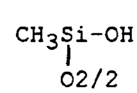

enthalten können, die in Mengen vorliegen, die 15 % der Summe p+q+r nicht übersteigen;
(ii) zyklischen Verbindungen gemäß der folgenden Durchschnittsformel (II):

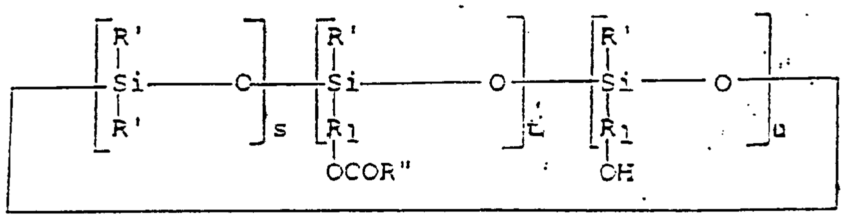

(II)

worin:

R′, R″ und $R_1$ die oben angegebenen Bedeutungen haben;

- s eine Zahl von 0 bis 20 ist;
- t eine Zahl von 1 bis 20 ist;
- u gleich Null oder gleich einer Zahl unterhalb oder gleich 0,5 t ist, wobei die Summe t+u 1 bis 20 beträgt, und

wobei die Summe s+t+u größer oder gleich 3 ist.

**2.** Zusammensetzung zum Rasieren der Haut, enthaltend in einem kosmetisch verträglichen wässrigen Medium, enthaltend ein Schaummittel, ein Polyorganosiloxan mit Acyloxyalkylfunktion, ausgewählt aus:

(i) linearen Verbindungen gemäß der folgenden Durchschnittsformel (I):

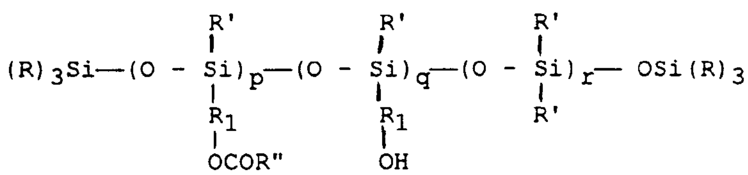

(I)

worin:

- die Reste R, gleich oder verschieden, unter Methyl-, Phenyl-, -OCOR″- und Hydroxylresten ausgewählt sind, wobei nur einer der Reste R pro Siliziumatom OH sein kann;
- die Reste R′, gleich oder verschieden, unter Methyl- und Phenylresten ausgewählt sind, wobei mindestens 60 Mol% aller Reste R und R′ Methylreste sind;
- $R_1$ eine zweiwertige lineare oder verzweigte Kohlenwasserstoff-Alkylenkette mit 2 bis 18 Kohlenstoffatomen, vorzugsweise 2 bis 6 Kohlenstoffatomen, ist, und insbesondere eine Trimethylenkette $-(CH_2)_3-$ und eine 2-Methyltrimethylenkette

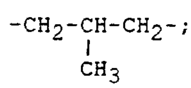

- R″ ein $C_{8-20}$-Alkyl- oder -Alkenylrest ist;
- r eine Zahl von 1 bis 120 ist;
- p eine Zahl von 1 bis 30 ist;
- q gleich Null oder eine Zahl unterhalb oder gleich 0,5 p ist, wobei die Summe p+q 1 bis 30 beträgt;
- wobei die Verbindung der Formel (I) gegebenenfalls Gruppierungen

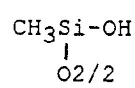

enthalten können, die in Mengen vorliegen, die 15 % der Summe p+q+r nicht übersteigen;

(ii) zyklischen Verbindungen gemäß der folgenden Durchschnittsformel (II):

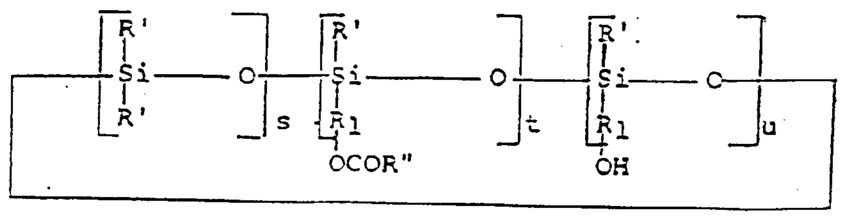

(II)

worin:

R', R" und $R_1$ die oben angegebenen Bedeutungen haben;

- s eine Zahl von 0 bis 20 ist;
- t eine Zahl von 1 bis 20 ist;
- u gleich Null oder gleich einer Zahl unterhalb oder gleich 0,5 t ist, wobei die Summe t+u 1 bis 20 beträgt, und

wobei die Summe s+t+u größer oder gleich 3 ist.

3. Zusammensetzung gemäß Anspruch 2, dadurch **gekennzeichnet**, daß in den Verbindungen der Formel (I) oder (II) $R_1$ eine lineare oder verzweigte zweiwertige Alkylenkohlenwasserstoffkette mit 2 bis 6 Kohlenstoffatomen darstellt.

4. Zusammensetzung gemäß einem der Ansprüche 2 bis 3, dadurch **gekennzeichnet**, daß in den Verbindungen der Formel (I) oder (II) R einen Methylrest, R' einen Methylrest, $R_1$ einen Trimethylenrest darstellen, R" die Gruppen $C_{12}H_{25}$, $C_{14}H_{29}$, $C_{16}H_{33}$,$C_{18}H_{37}$ und den Oleylrest bedeutet, r 1 bis 30, p 5 bis 16, q 0,5 bis 4 betragen und s+t+u 3 bis 10 beträgt.

5. Zusammensetzung gemäß jedem Anspruch 2 bis 4 dadurch **gekennzeichnet**, daß sie in einem kosmetish verträglichen Medium, enthaltend ein Schaummittel, ein Polyorganosiloxan der Formel (I) oder (II) in Mengenverhältnissen von 0,2 bis 3 Gew.%, vorzugsweise 0,5 bis 2 Gew.%, bezogen auf Gesamtgewicht der Zusammensetzung, enthält.

6. Zusammensetzung gemäß jedem Anspruch 2 bis 5, dadurch **gekennzeichnet**, daß sie in Form von Cremes, Gelem, selbstschäumenden Gelen, festen Seifestücken und Aerosolschäumen vorliegt.

7. Zusammensetzung gemäß jedem Anspruch 2 bis 6, dadurch **gekennzeichnet**, daß das kosmetisch verträgliche Medium aus Wasser zusammengesetzt ist und daß das Schaummittel eine Seife aus einer $C_{6-20}$-Fettsäure neutralisiert, durch ein alkalisches Mittel, oder ein synthetisches anionisches oberflächenaktives Mittel ist.

8. Zusammensetzung gemäß Anspruch 7, dadurch **gekennzeichnet**, daß das Schaummittel eine Seife aus einer Mischung von $C_{6-20}$-Fettsäuren und $C_{16-20}$-Fettsäuren ist, daß das Mengenverhältnis an $C_{6-20}$-Fettsäuren 10 bis 60 Gew.%, vorzugsweise 10 bis 25 Gew.%, bezogen auf Gesamtmenge der Seife, und das Mengenverhältnis an $C_{16-20}$-Fettsäuren 40 bis 90 Gew.%, vorzugsweise 75 bis 90 Gew.%, bezogen auf Gesamtmenge an Seife, betragen.

9. Zusammensetzung gemäß Anspruch 7, dadurch **gekennzeichnet**, daß das anionische oberflächenaktive Mittel ausgewählt ist aus Alkalimetall- und Ammoniumsalzen von Derivaten mit $C_{14-22}$-Fettsäureketten von Glutamin-, Isethion-, Sulfobernstein- und Sulfoessigsäuren, aus Sarcosin oder Taurin.

10. Zusammensetzung gemäß jedem der Ansprüche 2 bis 9, dadurch **gekennzeichnet**, daß sie 10 bis 85 Gew.% Wasser und 0,5 bis 80 Gew.% eines gemäß der Ansprüche 7 bis 9 definierten Schaummittels enthält.

11. Zusammensetzung gemäß jedem der Ansprüche 2 bis 10, dadurch **gekennzeichnet**, daß sie ein schäumendes Polymer oder ein von den Seifen und den in Anspruch 8 definierten Mitteln unterschiedenes nichtionisches, amphoteres oder anionisches schäumendes oberflächenaktives Mittel enthält.

12. Zusammensetzung gemäß jedem Anspruch 2 bis 11, dadurch **gekennzeichnet**, daß dis als Aerosol in Gegenwart eines Treibmittelgases zubereitet ist, um in Augenblick der Freisetzung ein selbstschäumendes Gel oder einen Schaum zu bilden.

13. Zusammensetzung gemäß Anspruch 12, dadurch **gekennzeichnet**, daß sie in Form eines Aerosolschaumes vorliegt, der 75 bis 85 Gew.% Wasser, 0,5 bis 15 Gew.% eines in den Ansprüchen 6 bis 8 definierten Schaummittels, 0,2 bis 3 Gew.% eines Organopolysiloxans der Formel (I) oder (II) und 2 bis 15 Gew.%, vorzugsweise 3 bis 10 Gew.%, eines Treibmittelgases enthält, bezogen auf Gesamtgewicht der Zusammensetzung.

14. Zusammensetzung gemäß jedem der Ansprüche 2 bis 13, dadurch **gekennzeichnet**, daß sie zusätzlich Feuchtigkeitsmittel, Transparenzmittel, filmbildende Mittel, weichmachende Mittel, Vernarbungsmittel, Mineralöle, Fettalkohole, Polymere, Verdickungsmittel, Stabilisierungsmittel, Beruhigungsmittel, anionische oberflächenaktive Mittel, die sich von den Seifen und den in Anspruch 8 definierten Mitteln unterscheiden, nicht-ionische oder amphotere Mittel oder deren Mischungen, Färbemittel, Konservierungsmittel, Antioxidantien und Parfüms enthält.

15. Verfahren zum Rasieren der Haut, dadurch **gekennzeichnet**, daß man auf dis Haut eine gemäß jedem der Ansprüche 2 bis 14 definierte Zusammensetzung aufträgt, die Haut mittels eines mechanischen Rasierers rasiert und mit Wasser spült.